# EUROPÄISCHE PATENTSCHRIFT

(11) **EP 1 834 615 B1**
(45) Veröffentlichungstag und Bekanntmachung des Hinweises auf die Patenterteilung: **15.08.2012**
(21) Anmeldenummer: 06005317.0
(22) Anmeldetag: 15.03.2006
(51) Int. Cl.: A61F 9/01

(54) **Steuerprogramm für die ophthalmologische Chirurgie**
Control program for ophthalmologic surgery
Programme de commande en chirurgie ophtalmique

(43) Veröffentlichungstag der Anmeldung: 19.09.2007
(73) Patentinhaber: WaveLight GmbH, 91058 Erlangen (DE)
(72) Erfinder: Mrochen, Michael, Dr., 8606 Nänikon (CH); Kittelmann, Olaf, Dr., 14163 Berlin (DE); Donitzky, Christof, 90542 Eckental (DE); Zatonski, Rafael, 90419 Nürnberg (DE)
(74) Vertreter: von Hellfeld, Axel

(56) Entgegenhaltungen:
- EP-A2- 1 138 291
- WO-A1-02/076320
- US-A1- 2002 193 704
- US-A1- 2004 243 112
- HEISTERKAMP A ET AL: "[Optimizing laser parameters for intrastromal incision with ultra-short laser pulses] OPTIMIERUNG DER LASERPARAMETER FUER DIE INTRASTROMALE SCHNITTFUEHRUNG MITTELS ULTRAKURZER LASERPULSE" OPHTHALMOLOGE, SPRINGER, BERLIN,, DE, Bd. 98, Nr. 7, Juli 2001 (2001-07), Seiten 623-628, XP002368976 ISSN: 0941-293X
- PERRY S. BINDER, MD: "Flap dimensions created with the IntraLase FS laser", JOURNAL OF CATERACT REFRACTIVE SURGERY, vol. 30, 2004, pages 26-32,

## Beschreibung

Die Erfindung betrifft ein Verfahren zum Erzeugen eines Steuerprogramms für die ophthalmologische Chirurgie, Steuerprogramme für die ophthalmologische Chirurgie, einen Rechner zum Steuern eines ophthalmologischen Lasersystems, und einen Datenträger mit Steuerprogrammen für die ophthalmologische Chirurgie.

Nachfolgend soll die Erfindung mit Blick auf das bekannte LASIK-Verfahren erläutert werden.

Bei der ophthalmologischen LASIK-Technik werden unerwünschte Abbildungseigenschaften des Auges durch Neuformung der Hornhaut beseitigt oder zumindest verringert.

Beim LASIK-Verfahren wird üblicherweise ein Deckelchen (sogenannter Flap) auf der Vorderseite der Hornhaut geformt und zur Seite geklappt. In einem zweiten Schritt wird im so freigelegten Stroma gemäß einem sogenannten Ablationsprofil Hornhautgewebe abgetragen. Sodann wird der Flap, der an einer Gelenkstelle (sogenanntes Hinge) mit der Kornea verbunden geblieben ist, zurückgeklappt und es tritt eine relativ schnelle Verheilung des Gewebes ein.

Zur Erzeugung des Flaps stehen zur Zeit im wesentlichen zwei Techniken zur Verfügung:

Zum einen wird mit einem sogenannten Mikrokeratom mechanisch eine oszillierende Schneide durch das Korneagewebe geführt, um den Flap zu formen.

Gemäß einem weiter fortgeschrittenen Verfahren wird auch zur Erzeugung des Flaps ein Laser eingesetzt. Hierzu dienen insbesondere FS-Laser (Femtosekundenlaser), deren Strahlung unterhalb der Oberfläche der Kornea im Stroma fokussiert wird um dort an einer Vielzahl von nebeneinander liegenden Stellen photodisruptiv eine Gewebetrennung zu bewirken. Dieser photodisruptive Prozess wird auch als LIOB (Laser-Induced Optical Breakdown) bezeichnet (vgl. Juhasz et al. "CORNEAL REFRACTIVE SURGERY WTTH FEMTOSECOND LASERS", IEEE Journal of Selected Topics in Quantum Electronics, Vol. 5, No. 4, July/August 1999). Wenn die Fokussierung der Laserpulse eine hinreichende Leistungsdichte (Energie pro Zeit- und Flächeneinheit) bewirkt, die über einem bestimmten Schwellenwert liegt, dann erfolgt der photodisruptive Prozess mit hinreichender Qualität, d.h. Glätte des Schnittes und Genauigkeit hinsichtlich der angestrebten Form. Um die hierfür erforderlichen hohen Intensitäten zu erreichen, werden in aller Regel ultrakurze Laserpulse benötigt, also Laserpulse im Femtosekundenbereich, wobei dieser Begriff üblicherweise Pulslängen geringer als eine Pikosekunde erfasst, also Pulslängen zwischen 1 Femtosekunde und 999 Femtosekunden. Die Fokussierung des Laserstrahls erfolgt dabei in der Regel im Mikrometerbereich.

Die US 2002/0193704 A1 offenbart ein Verfahren, ein System und eine Vorrichtung zur computergesteuerten Photodisruption von Augengewebe. Zum Erzeugen des Flaps wird dem Bediener eines Lasersystems eine Auswahl von Flap-Musterparametern zur Verfügung gestellt. Die Parameter sind mit Default-Werten initialisiert, die durch den Verwender geändert werden können. Unter Verwendung ausgewählter Flap-Musterparameter richtet die Software den Laserstrahl derart, dass er ein photodisruptives Verfahren bewirkt. Dem Nutzer wird ermöglicht, in einem Bedienfenster Flap-Parameter einzugeben. Ferner können Patientendaten, die beabsichtigte Behandlung und die Dicke der Komea eingegeben werden. Aus den eingegebenen Daten erzeugt eine Software des Lasersystems Werte für die X-, Y- und Z-Achse zum Fokussieren des Laserstrahls für das photodisruptive Verfahren.

Die US 2004/0243112 A1 betrifft die Abtragung von Augengewebe zur Korrektur einer Fehlsichtigkeit mittels der Femtosekunden-Lamellar-Keroplastik. Ferner werden verschiedene Ablationsmuster offenbart.

Die Erfindung setzt sich das Ziel, Mittel bereitzustellen, mit denen verbesserte ophthalmologisch-chirurgische Behandlungsergebnisse erzielbar sind.

Hierzu lehrt die Erfindung ein Verfahren zum Erzeugen eines Steuerprogramms für die ophthalmologische Chirurgie, insbesondere LASIK, mit dem ein gepulstes Lasersystem zum photodisruptiven Schneiden eines Flaps steuerbar ist, wobei das Programm mit folgenden Schritten gewonnen wird:
- Gewinnen von empirischen Daten, welche den Einfluss von Flapformen und Ablationsprofilen auf postoperative refraktive Ergebnisse beinhalten,
- Gewinnen von Messdaten bezüglich des zu behandelnden Auges,
- Berechnen einer Schnittform zum photodisruptiven Schneiden des Flaps unter Berücksichtigung der genannten empirischen Daten und der genannten Messdaten, und
- Erzeugen des Steuerprogramms auf Basis der berechneten Schnittform.

Die Erfindung macht sich also insbesondere die Erkenntnis zunutze, dass Flapformen häufig einen Einfluss haben auf das refraktive Behandlungsergebnis, also die Fähigkeit des behandelten Auges nach Änderung seiner lokalen Brechungseigenschaften, scharfe Abbildungen auf der Netzhaut bzw. der Fovea zu erzeugen. Bei LASIK-Operationen wird in der Regel ein sogenanntes Ablationsprofil mit bekannten Techniken berechnet. Die Erfindung passt insbesondere den Flapdurchmesser oder andere Abmessungen des Flaps (insbesondere Tiefe) an das Ablationsprofil an. Insbesondere bei myopen oder hyperopen Astigmatismen ist es von Vorteil, wenn der Flap in bestimmten Achsen einen hinreichenden Durchmesser aufweist, einschließlich der Übergangszone. Mit anderen Worten: Bei einer Vielzahl von Indikationen ist ein nicht rotationssymmetrischer (kreisförmiger) Flap optimal.

Der Erfindung liegt also die weitere Erkenntnis zugrunde, dass individuell angepasste Schnittformen bezüglich des Flaps hinsichtlich des refraktiven Endergebnisses nach der Verheilung des Flaps mit dem Stroma, bedeutsam sein können.

Deshalb beinhaltet die Erfindung auch ein Verfahren zum Erzeugen eines Steuerprogramm für die ophthalmologische Chirurgie, mit dem ein gepulstes Lasersystem zum photodisruptiven Schneiden eines Flaps steuerbar ist, wobei das Steuerprogramm für den Schnitt in unterschiedlichen Achsen unterschiedliche Flap-Durchmesser vorsieht.

Dabei stehen die beiden vorstehend genannten Achsen bevorzugt senkrecht aufeinander. Eine der Achsen verläuft üblicherweise (aber nicht immer) parallel zum sogenannten Gelenk (Hinge) des Flaps, also der Stelle, an welcher der Flap mit dem Korneagewebe verbunden bleibt. Dieses Gelenk könnte man auch als "Angel" oder "Scharnier" bezeichnen.

Eine weitere Variante der Erfindung lehrt Verfahren zum Erzeugen eines ein Steuerprogramm für die ophthalmologische LASIK-Chirurgie, mit dem ein gepulstes Lasersystem zum photodisruptiven Schneiden eines Flaps steuerbar ist, wobei das Steuerprogramm einen oder mehrere Vorsprünge in der Flapform vorsieht, mit denen der Flap nach seinem Rückklappen im Korneagewebe verankerbar ist.

Besondere Ausgestaltungen dieser Erfindungsvariante sehen zahnförmige oder hufeisenförmige Vorsprünge vor.

Die Erfindung lehrt das Gewinnen von empirischen Daten, welche den Einfluss von Flapformen und Ablationsprofilen auf das postoperative refraktive Ergebnis beinhalten. Dies heißt, dass die refraktiven Ergebnisse, welche bei unterschiedlichen Schnittformen schließlich gewonnen werden, im Rahmen von klinischen Studien evaluiert werden und anschließend daraus empirische Erkenntnisse gewonnen werden. Auf diese Weise können insbesondere optische Aberrationen höherer Ordnung durch eine geeignete Formgebung für den Flapdurchmesser minimiert werden, insbesondere durch eine nicht kreis-rotationssymmetrische Gestaltung des Flapumfanges (wobei diese Definitionen den Gelenk-Bereich des Flaps, der trivialer Weise nicht kreisrotationssymmetrisch ist, von der Betrachtung ausnehmen).

Eine weitere Variante der Erfindung lehrt ein Verfahren zum Erzeugen eines Steuerprogramms für die ophthalmologische LASIK-Chirurgie, wobei das Steuerprogramm vorsieht, dass der Flap-Durchmesser kleiner ist als das stromale Bett, in welches der Flap zurückzuklappen ist.

Die beiden vorstehend abgehandelten Erfindungsaspekte, also die Vorsprünge zur Verankerung und die Formung des Flapdurchmesser kleiner als das stromale Bett, in welches der Flap zurückgeklappt wird, gehen insbesondere das Problem des sogenannten Flap-Schrumpfens und der damit einhergehenden Faltenbildung an.

Eine Schrumpfung direkt nach dem Schnitt oder eine Verschiebung des Flaps durch biomechanische Effekte können zu höchst unerwünschten postoperativen Verwerfungen im Flap führen. Das Erzeugen von Fixationspunkten und -kanten mittels der genannten Vorsprünge wirkt solchen Verwerfungen entgegen. Weiterhin wird dadurch eine formgetreue Rückklappung des Flaps gefördert.

Im Unterschied zur Verwendung eines mechanischen Mikrokeratoms ermöglicht die Steuerung von Laserpulsen eine drei-dimensionale Formgebung für den Flap und, damit einhergehend, auch eine dreidimensionale Formgebung im Randbereich des verbleibenden Stroma-Bettes, welches bei der nachfolgenden Ablation unverändert bleiben kann, wenn die für die Fixation vorgegebenen Formgebungen außerhalb des refraktiven Ablationsprofils liegen.

Der Schrumpfungsprozess spielt in der Regel nur in der frühen postoperativen Phase eine Rolle. Nachdem der Flap rehydriert ist, ergibt sich insbesondere bei relativ hohen Myopiekorrekturen oder myopen Astigmatismen, dass der Flap für das verbliebene stromale Bett nicht passt. Deshalb sieht die Erfindung für diesen Fall ein Steuerprogramm vor, welches den Flapdurchmesser zumindest in bestimmten Bereichen kleiner macht als der zugehörige Durchmesser des stromalen Bettes.

Die Erfindung beinhaltet auch einen mit den beschriebenen Steuerprogrammen programmierten Rechner, der ein gepulstes Lasersystem in an sich bekannter Weise steuert, zum Beispiel mit dem Galvanometerprinzip.

Nachfolgend werden Ausführungsbeispiele der Erfindung anhand der Zeichnung näher erläutert. Es zeigt bzw. zeigen:
- Figur 1: schematisch eine Vorrichtung für die ophthalmologische Chirurgie, insbesondere LASIK,
- Figuren 2 bis 5: schematisch Ausführungsbeispiele für Flap-Schnittformen;
- Figuren 6 bis 9: schematisch Ausführungsbeispiele für Flap-Schnittformen mit Verankerungsmitteln.

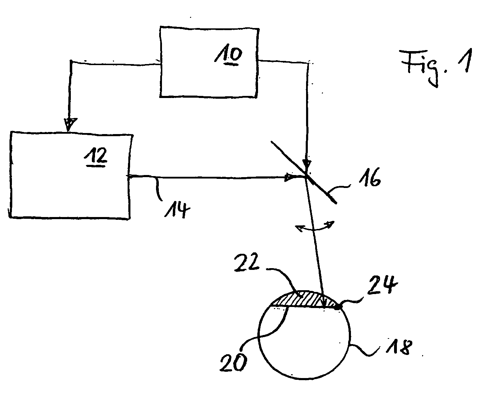

Figur 1 zeigt ein System für die ophthalmologische LASIK-Chirurgie mit einem Rechner 10, und einem Laser 12, der einen im Femtosekundenbereich gepulsten Laserstrahl 14 emittiert. Der Rechner 10 steuert sowohl den Laser 12 als auch einen galvanisch bewegbaren Spiegel 16, mit dem der Laserstrahl auf das zu behandelnde Auge 18 gerichtet wird. Der Laserstrahl wird in an sich bekannter Weise an der Stelle fokussiert, wo der Schnitt 20 zur Erzeugung des Flaps 22 geführt wird. Durch den oben beschriebenen photodisruptiven Effekt wird dann der Flap 22 geformt, der über ein Gelenk 24 mit dem Korneagewebe verbunden bleibt. Dies ist als solches Stand der Technik.

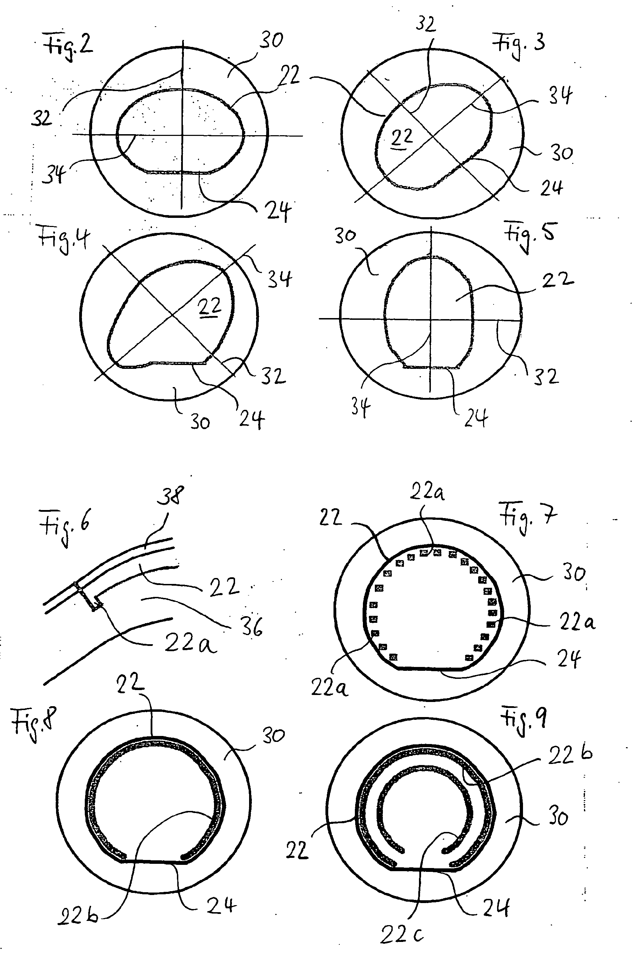

Die Figuren 2 bis 5 zeigen individuelle Flapformen, in Abhängigkeit von individuellen Indikationen.

Die Figuren 2 bis 5 zeigen jeweils eine Kornea 30 in Draufsicht. Die Umrisse des Flaps sind mit dem Bezugszeichen 22 bezeichnet. Das Gelenk 24 ist in der Regel geradlinig, wie jeweils in den Figuren 2 bis 5 zu erkennen ist.

Weiterhin zeigen die Figuren 2 bis 5 zueinander senkrecht stehende Achsen, nämliche eine steile Achse 32 (steep axis) und eine flache Achse 34 (flat axis). Die steile Achse entspricht der Richtung über den Flap, in welcher steilere (stärkere) Krümmungen vorliegen, die flache Achse die Richtung über den Flap, in welcher schwächere Krümmungen vorliegen.

Die Figuren 2 bis 5 betreffen eine Astigmatismus-Korrektur mit individuellen Flaps. Figur 2 zeigt eine flache Achse in horizontaler Richtung bei einem Inferior-Gelenk (Hinge). Bei Figur 3 ist die flache Achse (34) um 30°+ gedreht, ebenso das Gelenk 24. Beim Beispiel gemäß Figur 4 liegt wieder ein Inferior-Gelenk vor und die flache Achse ist um 30°+ gedreht. Figur 5 zeigt schließlich wiederum ein Inferior-Gelenk mit einer flachen Achse, die vertikal verläuft und senkrecht auf dem Gelenk 24 steht.

Die Figuren 2 bis 5 zeigen typische individuell angepasste Flapformen, die bei bestimmten, empirisch ermittelten Indikationen und zugehörigen entsprechenden Ablationsprofilen ein optimales Refraktionsergebnis nach der Verheilung liefern.

Eingabeparameter bei diesem Verfahren sind insbesondere biometrische Daten der Hornhaut des Epithels (Dickenverteilung) und des vorderen Augenabschnittes (z.B. Vorderkammertiefe, Hornhautbrechkraft etc.). Weiterhin sind typische Eingabeparameter biomechanische Messergebnisse mit unterschiedlichen Messverfahren, zum Beispiel die Poisson-Konstante, der Young's Module, sowie Daten über das elastische, insbesondere viskoelastische Verhalten der Hornhaut. Insbesondere betreffen empirisch gewonnene Daten die Epithelglättung im postoperativen Verlauf und auch das verwendete Ablationsprofil.

Ausgabedaten, d.h. die "gewonnenen empirischen Daten" betreffen Variationen des Durchmessers und der Dicke des Flaps in Abhängigkeit vom genannten Ablationsprofil. Dies hat insbesondere das Ziel durch die Operation induzierte oder bestehende optische Aberrationen zu minimieren. Weitere Ausgabedaten sind eine mathematische oder eine diskrete Beschreibung des Flaps in einer Matrix mit drei Raumkoordinaten und eine Vorgabe für den zeitlichen Ablauf des Schnittes zur Minimierung von Aberrationen. Bevorzugt werden die Daten in einem Nomogramm berücksichtigt.

Figur 6 zeigt eine besondere Ausgestaltung eines Flaps 22 mit einem zahnförmigen Vorsprung 22a zur Verankerung im Stroma 36 der Kornea. Dargestellt ist weiterhin das Epithel 38. Figur 6 zeigt einen Schnitt durch einen Teilbereich der Kornea, in dem der Verankerungszahn 22a wirkt. Figur 7 zeigt eine Draufsicht auf die gleiche Kornea 30 mit einer Vielzahl von Zähnen 22a, die sich entlang dem Umfang des Flaps 22 erstrecken, mit Ausnahme des Bereichs des Gelenks 24, bei dem keine Verankerung erforderlich ist, weil das Korneagewebe integral bleibt. Die Figuren 8 und 9 zeigen jeweils eine Draufsicht auf eine Kornea 30 und einen Flap 22. Beim Ausführungsbeispiel nach Figur 8 ist ein etwa hufeisenförmiger Vorsprung 22b vorgesehen, der im Schnitt so aussähe, wie der zahnförmige Vorsprung 22a gemäß Figur 6. Es erfolgt also mit dem hufeisenförmigen Vorsprung 22b gemäß Figur 8 eine umlaufende Verankerung am Rande des Flaps 22 im Stroma-Bett. Beim Ausführungsbeispiel gemäß Figur 9 sind zwei konzentrische hufeisenförmige Verankerungsvorsprünge 22b, 22c vorgesehen.

## Patentansprüche

1. Verfahren zum Erzeugen eines Steuerprogramms für die ophthalmologische LASIK-Chirurgie, mit dem ein gepulstes Lasersystem (12, 14, 16) zum photodisruptiven Schneiden eines Flaps steuerbar ist, mit folgenden Schritten:
- Gewinnen von empirischen Daten, welche den Einfluss von Flapformen und Ablationsprofilen auf postoperative refraktive Ergebnisse beinhalten, und
- Gewinnen von Messdaten bezüglich des zu behandelnden Auges,
- Berechnen einer optimalen Schnittform zum photodisruptiven Schneiden des Flaps unter Berücksichtigung der genannten empirischen Daten und der genannten Messdaten und
- Erzeugen des Steuerprogramms auf Basis der berechneten Schnittform.

2. Verfahren zum Erzeugen eines Steuerprogramms für die ophthalmologische LASIK-Chirurgie nach Anspruch 1, wobei mit dem Steuerprogramm ein gepulstes Lasersystem zum photodisruptiven Schneiden eines Flaps steuerbar ist, **dadurch gekennzeichnet, dass** das Steuerprogramm für den Schnitt in unterschiedlichen Achsen (32, 34) unterschiedliche Flap-Formen, inbesondere Flap-Durchmesser und/oder Flap-Tiefen, vorsieht.

3. Verfahren zum Erzeugen eines Steuerprogramms für die ophthalmologische LASIK-Chirurgie nach Anspruch 2, **dadurch gekennzeichnet, dass** die Achsen (32, 34) zueinander senkrecht stehen.

4. Verfahren zum Erzeugen eines Steuerprogramms für die ophthalmologische LASIK-Chirurgie nach einem der Ansprüche 2 oder 3, **dadurch gekennzeichnet, dass** eine der Achsen im wesentlichen senkrecht steht auf dem Gelenk (24) des Flaps.

5. Verfahren zum Erzeugen eines Steuerprogramms für die ophthalmologische LASIK-Chirurgie nach Anspruch 1, wobei mit dem Steuerprogramm ein gepulstes Lasersystem zum photodisruptiven Schneiden eines Flaps steuerbar ist, **dadurch gekennzeichnet, dass** das Steuerprogramm einen oder mehrere Vorsprünge (22a, 22b, 22c) in der Flapform vorsieht, mit denen der Flap (22) nach seinem Rückklappen im Korneagewebe (36) verankerbar ist.

6. Verfahren zum Erzeugen eines Steuerprogramms für die ophthalmologische LASIK-Chirurgie nach Anspruch 5, **dadurch gekennzeichnet, dass** Vorsprünge (22a) zahnförmig sind.

7. Verfahren zum Erzeugen eines Steuerprogramms für die ophthalmologische LASIK-Chirurgie nach Anspruch 5, **dadurch gekennzeichnet, dass** ein Vorsprung (22b, 22c) die Form eines nicht geschlossenen Ringes hat.

8. Verfahren zum Erzeugen eines Steuerprogramms für die ophthalmologische LASIK-Chirurgie nach Anspruch 1, **dadurch gekennzeichnet, dass** das Steuerprogramm vorsieht, dass der Flap-Durchmesser kleiner ist als das stromale Bett, in welches der Flap zurückzuklappen ist.

9. Rechner, programmiert mit einem Steuerprogramm erhalten durch das Verfahren gemäß einem der Ansprüche 1 bis 8.

10. Datenträger mit einem Steuerprogramm erhalten durch das Verfahren gemäß einem der Ansprüche 1 bis 8.

## Claims

1. Method for generating a control program for ophthalmologic LASIK surgery, with which a pulsed laser system (12, 14, 16) can be controlled for the photodisruptive cutting of a flap, having the following steps:
- obtaining empirical data, which relate to the effect of flap shapes and ablation profiles on postoperative refractive results,
- obtaining measurement data relating to the eye to be treated,
- calculating an optimal cutting shape for the photo-disruptive cutting of the flap by taking into account the said empirical data and the said measurement data, and
- generating the control program on the basis of the calculated cutting shape.

2. Method for generating a control program for ophthalmologic LASIK surgery according to claim 1, wherein, by means of the controlled program a pulsed laser system for photo-disruptive cutting of a flap can be controlled, **characterized in that** the control program provides for different flap shapes, in particular different flap diameters and/or different flap strengths, for cutting along different axes (32, 34).

3. Method for generating a control program for ophthalmologic LASIK surgery according to claim 2, **characterized in that** the axes (32, 34) are mutually perpendicular.

4. Method for generating a control program for ophthalmologic LASIK surgery according to one of claims 2 or 3, **characterized in that** one the axes is essentially perpendicular to the hinge (24) of the flap.

5. Method for generating a control program for ophthalmologic LASIK surgery according to claim 1, with which a pulsed laser system can be controlled for the photo-disruptive cutting of a flap, **characterized in that** the control program provides one or more projections (22a, 22b, 22c) in the flap shape, by which the flap (22) can be anchored in the corneal tissue (36) after it is folded back.

6. Method for generating a control program for ophthalmologic LASIK surgery according to claim 5, **characterized in that** the projections (22a) are toothshaped.

7. Method for generating a control program for ophthalmologic LASIK surgery according to claim 5, **characterized in that** a protrusion (22b, 22c) has the shape of an unclosed ring.

8. Method for generating a control program for ophthalmologic LASIK surgery according to claim 1, **characterized in that** the control program provides that the flap-diameter is smaller than the stromal bed in which the flap can be folded back.

9. Computer programmed with a control program obtained by a method according to one of the claims 1 to 8.

10. Data carrier comprising a control program obtained by the method in accordance with one of the claims 1 to 8.

## Revendications

1. Procédé pour créer un programme de commande en chirurgie ophtalmologique LASIK permettant la commande d'un système laser pulsé (12, 14, 16) destiné à la coupe photodisruptive d'un volet cornéen, lequel procédé comprenant les étapes suivantes :
- obtention de données empiriques quant à l'influence des formes du volet et des profils d'ablation sur les résultats réfractifs postopératoires et
- obtention de données de mesure relatives à l'oeil à traiter,
- calcul d'une forme de coupe optimale pour la coupe photodisruptive du volet compte tenu desdites données empiriques et desdites données de mesure et
- création du programme de commande sur la base de la forme de coupe calculée.

2. Procédé pour créer un programme de commande en chirurgie ophtalmologique LASIK selon la revendication 1, le programme de commande permettant la commande d'un système laser pulsé destiné à la coupe photodisruptive d'un volet cornéen, **caractérisé en ce que** le programme de commande prévoit différentes formes de volet, en particulier différents diamètres et/ou profondeurs de volet pour la coupe dans différents axes (32, 34).

3. Procédé pour créer un programme de commande en chirurgie ophtalmologique LASIK selon la revendication 2, **caractérisé en ce que** les axes (32, 34) sont perpendiculaires l'un par rapport à l'autre.

4. Procédé pour créer un programme de commande en chirurgie ophtalmologique LASIK selon l'une des revendications 2 ou 3, **caractérisé en ce qu'**un des axes est pour l'essentiel perpendiculaire sur l'articulation (24) du volet.

5. Procédé pour créer un programme de commande en chirurgie ophtalmologique LASIK selon la revendication 1, le programme de commande permettant la commande d'un système laser pulsé destiné à la coupe photodisruptive d'un volet cornéen, **caractérisé en ce que** le programme de commande prévoit une ou plusieurs parties saillantes (22a, 22b, 22c) dans la forme du volet au moyen desquelles le volet (22), une fois rabattu, peut être ancré dans le tissu cornéen (36).

6. Procédé pour créer un programme de commande en chirurgie ophtalmologique LASIK selon la revendication 5, **caractérisé en ce que** les parties saillantes (22a) sont en forme de dents.

7. Procédé pour créer un programme de commande en chirurgie ophtalmologique LASIK selon la revendication 5, **caractérisé en ce qu'**une partie saillante (22b, 22c) a la forme d'un anneau non fermé.

8. Procédé pour créer un programme de commande en chirurgie ophtalmologique LASIK selon la revendication 1, **caractérisé en ce que** le programme de commande prévoit que le diamètre du volet est plus petit que le lit stromal dans lequel est rabattu le volet.

9. Ordinateur programmé à l'aide d'un programme de commande obtenu par le procédé selon les revendications 1 à 8.

10. Support de données comportant un programme de commande obtenu à l'aide du procédé selon l'une des revendications 1 à 8.
